# EUROPÄISCHE PATENTANMELDUNG

(11) **EP 0 902 092 A2**
(43) Veröffentlichungstag der Anmeldung: **17.03.1999**
(21) Anmeldenummer: 98117436.0
(22) Anmeldetag: 15.09.1998
(51) Int. Cl.: C12Q 1/68

(54) **Verfahren zur identifizierung von zielgenen von transkriptionsfaktoren**

(30) Priorität: 15.09.1997 DE 19740578
(71) Anmelder: GSF-Forschungszentrum für Umwelt und Gesundheit GmbH, 85764 Oberschleissheim (DE); CENTRE NATIONAL DE LA RECHERCHE SCIENTIFIQUE (CNRS), 75794 Paris Cédex 16 (FR)
(72) Erfinder: Wurst, Wolfgang, Dr., 80809 München (DE); Prochiantz, Alain, Dr., 75006 Paris (FR)
(74) Vertreter: Reinhard - Skuhra - Weise & Partner

(57) **Zusammenfassung**

Erfindungsgemäß wird ein Verfahren zur Identifizierung von Transkriptionsfaktor responsiblen Elementen, Zielgenen und/oder Cofaktoren von Transkriptionsfaktoren mit den nachfolgenden Schritten bereitgestellt:
(a) Einbringen eines Genfallenvektors in eine eukaryontische Zelle in Kultur, wobei der Genfallenvektor zumindest, in funktioneller Anordnung, die nachfolgenden Elemente aufweist:
   - ein Reportergen
   - eine Polyadenylierungsstelle
   - ein Selektionsmarkergen
(b) Selektion auf Zellen, die den Vektor enthalten;
(c) in Kontakt bringen der selektierten Zellen mit einem Transkriptionsfaktor, dessen zugehöriges Transkriptionsfaktor responsible Element, Zielgen und/oder zugehöriger Cofaktor analysiert werden soll;
(d) Identifizieren und Kultivieren solcher Zellen, die eine Veränderung der Reportergenaktivität zeigen;
(e) Identifizieren der Zielgene und/oder Cofaktoren der Transkriptionsfaktoren und/oder der Transkriptionsfaktor responsiblen Elemente.

## Beschreibung

Die Erfindung betrifft ein Verfahren zur Identifizierung von Transkriptionsfaktor responsiblen Elementen, Zielgenen und/oder Cofaktoren von Transkriptionsfaktoren.

Unter den zahlreichen Genen, die die Metazoenentwicklung steuern, spielen die Homöogene eine Schlüsselrolle bei der Entscheidung für einen Zellstammbaum und bei der Etablierung der morphologischen Identität multizellulärer Domänen. Sie werden in allen Zellschichten des Organismus und zu verschiedenen Stadien während der Entwicklung exprimiert. Sie codieren für Homöoproteine, eine große Familie von Transkriptionsfaktoren, die durch eine hochkonservierte, 60 Aminosäuren lange DNA-bindende Domäne, die Homöodomäne, gekennzeichnet ist.

Die als "Engrailed" bezeichneten Säugetier-Homöoproteine, Engrailed-1 und Engrailed-2 (En1 und En2), werden zuerst im anterioren Neuroepithel im 1-Somiten-Stadium (im Fall von En1) und im 5-Somiten-Stadium (im Fall von En2) in einer Domäne, die sich später in das Mesencephalon- und Rhombencephalon-Gebiet entwickelt, exprimiert. Danach wird *En1* im Rückenmark im Dermomyotom der Somiten und im ventralen Ektoderm der Extremitätenanlage exprimiert. Geninaktivierungsexperimente zeigten, daß *En1* für die Entwicklung des Mittel-/Rautenhirns, des Sternums, und der ventralen Extremitätenanlage notwendig ist, wohingegen *En2*-Mutanten nur einen leichten Mittel-/Rautenhirn-Musterbildungsphänotyp zeigen.

Genaustauschexperimente von *En1* durch *En2* deuten jedoch darauf hin, daß die beiden Proteine identische biochemische Eigenschaften besitzen und daß die verschiedenen Phänotypen, die bei der Hirnentwicklung bei *En2*- und *En1*-Mutanten beobachtet werden, aufgrund zeitlicher Unterschiede in der Expression der beiden Gene während der frühen Neurogenese zustande kommen. Daher werden in dieser Studie *En1* und *En2* häufig zusammengefaßt und als Engrailed bezeichnet.

Wie am Beispiel von *Engrailed* beschrieben, waren Analysen der Expressionsmuster und der morphologischen Modifikationen, die in Mäuseembryos mit Mutationen in bestimmten Homöogenen erzeugt wurden, von unschätzbarem Wert für das Verständnis der Funktion dieser Homöogene während der Entwicklung. Für ein Verständnis ihrer genauen Wirkungsweise müssen die genetischen Netzwerke, die ihre Funktion ausüben, jedoch identifiziert werden. Betrachtet man die Phänotypen von *Engrailed*-Mutanten, sind potentielle Ziele an der Gewebemusterbildung (sezernierte Moleküle), der Zelltypbestimmung (andere Transkriptionsfaktoren), der Zellwanderung (Zell- und Substratadhäsionsmoleküle) und der Zellmorphogenese (Strukturproteine) beteiligt.

Bei *Drosophila* konnten mit genetischen Ansätzen, die die funktionellen Wechselwirkungen in Mutanten aufdeckten, und durch Suche nach DNA-Bindungsstellen in Polytänchromosomen die Zielgene jedes gegebenen Transkriptionsfaktors gesucht werden. Die Anwendung dieser Techniken führte zur Identifizierung einer Reihe mutmaßlicher Zielgene von *engrailed*, wie *hedgehog*, *decapentaplegic*, *cubitus-interruptus* (*ci*), *polyhometi*c und *β3-Tubulin*. Nur von *ci* und β*3-Tubulin* konnte gezeigt werden, daß sie direkte Zielgene von *engrailed* sind. Die bei der Fruchtfliege verwendeten genetischen Verfahren, die im Prinzip auch Zugang zu genetischen Reaktionswegen gewähren sollten, sind bei Vertebraten aufgrund der sehr großen Anzahl an Nachkommen, die untersucht werden müßten und wegen des Fehlens von Polytänchromosomen nicht so einfach anwendbar. Bei Vertebraten wurden die meisten möglichen Homöoprotein-Zielgene entweder zufällig durch Promotoranalysen oder durch Homologie mit bereits in Invertebraten, hauptsächlich in *Drosophila*, identifizierten Genen entdeckt. In den meisten Fällen war der Nachweis, daß die identifizierten Gene wirkliche Zielgene sind, ziemlich umständlich. Von besonderer Bedeutung ist, daß mit Ausnahme einiger, durch eine kürzliche NCAM-Studie dargestellter Ausnahmen von fast keinem dieser Zielgene gezeigt wurde, daß es in vivo durch Homöoproteine reguliert wird.

Es ist eine Aufgabe der vorliegenden Erfindung, ein Verfahren zur Identifizierung von Transkriptionsfaktor responsiblen Elementen, Zielgenen und/oder Cofaktoren von Transkriptionsfaktoren bereitzustellen, das eine systematische Bestimmung dieser Elemente, Zielgene und Cofaktoren ermöglicht und die aus dem Stand der Technik bekannten Nachteile vermeidet.

Diese Aufgabe wird erfindungsgemäß durch ein Verfahren zur Identifizierung von Transkriptionsfaktor responsiblen Elementen, Zielgenen und/oder Cofaktoren von Transkriptionsfaktoren mit den nachfolgenden Schritten gelöst:
(a) Einbringen eines Genfallenvektors in eine eukaryontische Zelle in Kultur, wobei der Genfallenvektor zumindest, in funktioneller Anordnung, die nachfolgenden Elemente aufweist:
   - ein Reportergen
   - eine Polyadenylierungsstelle
   - ein Selektionsmarkergen
(b) Selektion auf Zellen, die den Vektor enthalten;
(c) in Kontakt bringen der selektierten Zellen mit einem Transkriptionsfaktor, dessen zugehöriges Transkriptionsfaktor responsible Element, Zielgen und/oder zugehöriger Cofaktor analysiert werden soll;
(d) Identifizieren und Kultivieren solcher Zellen, die eine Veränderung der Reportergenaktivität zeigen;
(e) Identifizieren der Zielgene und/oder Cofaktoren der Transkriptionsfaktoren und/oder der Transkriptionsfaktor responsiblen Elemente.

Genfallenvektoren sind an sich bekannt und wurden beispielsweise in Hill und Wurst, 1993a und Hill und Wurst, 1993b, beschrieben. Auf diese Veröffentlichung wird vollinhaltlich Bezug genommen.

Die erfindungsgemäß verwendbaren Genfallenvektoren enthalten zumindest ein Reportergen, eine Polyadenylierungsstelle und ein Selektionsmarkergen, die in funktioneller Verbindung angeordnet sind.

Die Transkription des Reportergens wird nach Integration des Genfallenvektors in das Genom einer eukaryontischen Zelle dann modifiziert, wenn es, nach Zugabe eines Transkriptionsfaktors, unter der Kontrolle eines Transkriptionsfaktor responsiblen Elements steht. Unter Modifikation wird erfindungsgemäß eine Induktion oder Repression der Transkription des Reportergens verstanden, d.h. eine Erhöhung oder Absenkung der Transkriptionsrate. Als Reportergen können beliebige, in eukaryontischen Zellen nachweisbare Gene verwendet werden. Derartige Gene sind beispielsweise das LacZ-Gen, das Luciferasegen, das Green-Fluoreszenz-Protein-Gen und das CAT-Gen. Dem Fachmann sind weitere verwendbare Reportergene bekannt, die in Abhängigkeit von der Zielzelle und vom zu untersuchenden Transkriptionsfaktor ausgewählt werden.

Zur Expression wird weiterhin eine Polyadenylierungsstelle benötigt, die in funktioneller Verbindung mit dem Reportergen und eventuell weiteren, auf dem Vektor angeordneten Genen vorliegt.

Um diejenigen Zellen zu identifizieren, die nach dem Einbringen des Genfallenvektors diesen in integrierter Form enthalten, wird zusätzlich ein Selektionsmarkergen eingebaut. Das Selektionsmarkergen kann ein beliebiges, in eukaryontischen Zellen selektierbares Gen sein, beispielsweise ein Antibiotikumresistenzgen, beispielsweise ein Neomyzin- oder Hygromycin-Resistenzgen.

Auf dem Genfallenvektor kann ein Promotor für das Selektionsmarkergen angeordnet sein, der in der gewählten Zielzelle aktiv ist. Mögliche Promotoren sind der Phosphoglyceratkinasepromotor, der Cytomegalieviruspromotor oder der β-Aktin-Promotor.

In einer weiteren Ausführungsform der Erfindung wird ein Fusionsprotein zwischen dem Reportergen und dem Resistenzmarkergen ausgebildet; in diesen Fällen trägt weder das Resistenzmarkergen noch das Selektionsmarkergen einen eigenen Promotor, sondern die Expression der entsprechenden Genprodukte wird nach Integration in ein Gen, das in den Zielzellen exprimiert wird, von zelleigenen Promotoren gesteuert.

Bei Verwendung eines Genfallenvektors, der keinen eigenen Promoter für das Reportergen und das Resistenzmarkergen trägt, können Integrationen in aktive Gene erkannt werden. Beispielsweise enthält ein derartiger Vektor eine Spleißakzeptorstelle, das Reportergen, das Resistenzmarkergen und eine Polyadenylierungsstelle. Ein Beispiel für das Markergen ist das lacZ-Gen, ein Beispiel für das Resistenzgen, das Neomycinresistenzgen.

Das Einbringen des Genfallenvektors in die Zielzelle erfolgt durch beliebige, aus dem Stand der Technik bekannte Methoden. Beispiele hierfür sind Transfektionsverfahren, Lipofektionsverfahren, Elektroporation und Verfahren unter Anwendung retroviraler Vektoren.

Der Genfallenvektor kann weitere Gene, Regulations- und Kontrollsequenzen etc. enthalten, um die Expression der auf dem Vektor befindlichen Gene und seine Integration in das Genom der Zielzelle sowie seine Replikation sicherzustellen. Im einzelnen wird hier auf die oben genannte Literatur von Hill und Wurst, 1993a und Hill und Wurst, 1993b, hingewiesen.

Der Genfallenvektor wird in eine Zielzelle eingebracht, wobei die Auswahl der Zielzelle vom zu untersuchenden Transkriptionsfaktor responsiblen Element, vom zu untersuchenden Zielgen des Transkriptionsfaktors und vom zu untersuchenden Cofaktor des Transkriptionsfaktors abhängt. Bevorzugte Zielzellen sind beispielsweise embryonale Stammzellinien, Fibroblasten-Zellinien, neuronale Zellinien, Tumorzellinien und Hefezellen.

Das Einbringen des Genfallenvektors in eine embryonale Stammzellinie hat den Vorteil, daß diese zur Erzeugung von chimären Säugetieren einsetzbar ist. Durch Kreuzen solcher Chimären mit Wildtyp-Tieren kann die durch die Integration hervorgerufene Mutation auf die nächste Generation übertragen werden. Dadurch kann zusätzlich zur Identifikation eines Zielgenes dessen Funktion im Gesamtorganismus studiert werden.

Nach Einbringen des Genfallenvektors in die eukaryontische Zellen werden diese unter Selektionsbedingungen gehalten, um diejenigen Zellen zu selektieren, die den Genfallenvektor in integrierter Form enthalten. In einer bevorzugten Ausführungsform der Erfindung enthält der Genfallenvektor weiterhin ein Reportergen zur Negativselektion, um beispielsweise gegen Integrationen in konstitutiv aktive Gene zu selektieren. Als Reportergen wird beispielsweise das Thymidinkinasegen, das Diphteriegen oder das HAT-Gen in den Genfallenvektor insertiert. Bei Verwendung des Thymidinkinasegens als Reportergen zur Selektion gegen Integrationen in konstitutiv aktive Gene werden die Zellen Gancyclovir ausgesetzt. Zellen, die Thymidinkinase exprimieren, werden hierdurch ausgeschaltet.

In einem nächsten Verfahrensschritt nach Selektion auf solche Zellen, die den Vektor enthalten, werden die selektierten Zellen mit einem Transkriptionsfaktor in Kontakt gebracht. Der Transkriptionsfaktor wird entweder zum Zellkulturmedium zugegeben oder das für den Transkriptionsfaktor codierende Gen liegt auf einem Plasmid in der Zelle vor, entweder in integrierter oder nicht integrierter Form, und befindet sich unter der Kontrolle eines induzierbaren Promotors.

Wird der Transkriptionsfaktor dem Zellkulturmedium zugegeben, muß er in der Lage sein, die Zellmembran zu passieren, um in den Zellkern einzudringen. In einer besonders bevorzugten Ausführungsform der Erfindung enthält der Transkriptionsfaktor eine Homeodomäne, die ihn befähigt, die Zellmembran zu passieren. Die dritte Helix von Homeoproteinen ist hierzu insbesondere geeignet. Die Domäne umfaßt 60 Aminosäuren und wird nachfolgend näher beschrieben. Selbstverständlich sind auch weiter Domänen einsetzbar, die das Protein zum Passieren der Zellmembran befähigen. Enthält der zu untersuchende Transkriptionsfaktor keine Sequenzen, die ihn zur Passage der Zellmembran befähigen, können diese an einer geeigneten Stelle an das Transkriptionsfaktorprotein angehängt werden.

Das für den Transkriptionsfaktor kodierende Gen wird beispielsweise ausgewählt aus der Gruppe, umfassend Zinkfingergene, Pou-Gene, Homeobox enthaltende Gene, HGM-Box-enthaltende Gene oder Winged-Helix-Gene.

Falls der Genfallenvektor in ein Gen insertierte, das unter der Kontrolle eines Transkriptionsfaktor responsiblen Elementes steht, wird, nach Zugabe und Bindung des Transkriptionsfaktors an dieses Element, das auf dem Genfallenvektor befindliche Reportergen exprimiert werden. Die Expression des Reportergens kann in an sich bekannter Weise nachgewiesen werden. Handelt es sich bei dem Reportergen um das LacZ-Gen, wird, wie in Hill und Wurst, 1993, beschrieben, auf β-Galaktosidase-Aktivität gefärbt. Der Nachweis der verwendeten Reportergene ist aus dem Stand der Technik bekannt.

Diejenigen Zellen, die eine Veränderung der Reportergenaktivität zeigen, werden identifiziert und kultiviert. Durch an sich bekannte Methoden können dann die Zielgene der Transkriptionsfaktoren, die Cofaktoren der Transkriptionsfaktoren oder die Transkriptionsfaktor responsiblen Elemente identifiziert und analysiert werden.

Das erfindungsgemäße Verfahren wird nachfolgend beispielhaft anhand der Identifizierung von Genen im genetischen Regulationsweg der Homöoboxgene von Säugetieren gezeigt. Hierbei wird ein Induktionsgenfallenansatz in embryonalen Stammzellen (ES) mit der Translokation von Homöopeptiden über biologische Membranen kombiniert. Diese Ausführungsform der Erfindung macht sich zu Nutze, daß verschiedene Homöodomänen und Homöoproteine über die Zellmembran translozieren, wenn sie zum Kulturmedium zugegeben werden, sodaß sie schließlich in den Zellkern gelangen. Dort können sie die Transkription eines Reportergens unter der Kontrolle eines Homöoprotein-responsiblen Elementes regulieren, was zu einer Modulierung des Neuritenwachstums führen kann.

Die nachfolgenden Abläufe in der Zelle erfordern es, daß die spezifischen DNA-Bindungseigenschaften einer internalisierten Homöodomäne beibehalten werden; dies zeigt, daß die translozierten Homöodomänen mit der Transkriptionsaktivität der endogenen Homöoproteine in Wechselwirkung treten und daher zur Identifikation von unter der Kontrolle der Homöoproteine stehenden Genen verwendbar sind. Durch Verwendung einer exogen zugegebenen Homöodomäne als Induktor für die Durchmusterung einer Bank mit Genfallenintegrationen in embryonalen Stammzellen (ES) kann man so Gene im genetischen Regulationsweg von Homöoproteinen, einschließlich direkter Homöoprotein-Zielgene, identifizieren. Da En2 in undifferenzierten ES-Zellen exprimiert wird, wurde beispielhaft diese neue, leistungsfähige Induktionsgenfallentechnologie bei ES-Zellen angewandt, um nach Zielgenen zu suchen, die im genetischen Regulationsweg von Engrailed aktiv sind. Unter Verwendung der En2-Homöodomäne (EnHD) als chemischen Induktor haben wir 30000 Genfallenvektorintegrationen durchmustert und den *dt/BPAG1*-Locus als Ziel von Engrailed-Proteinen identifiziert.

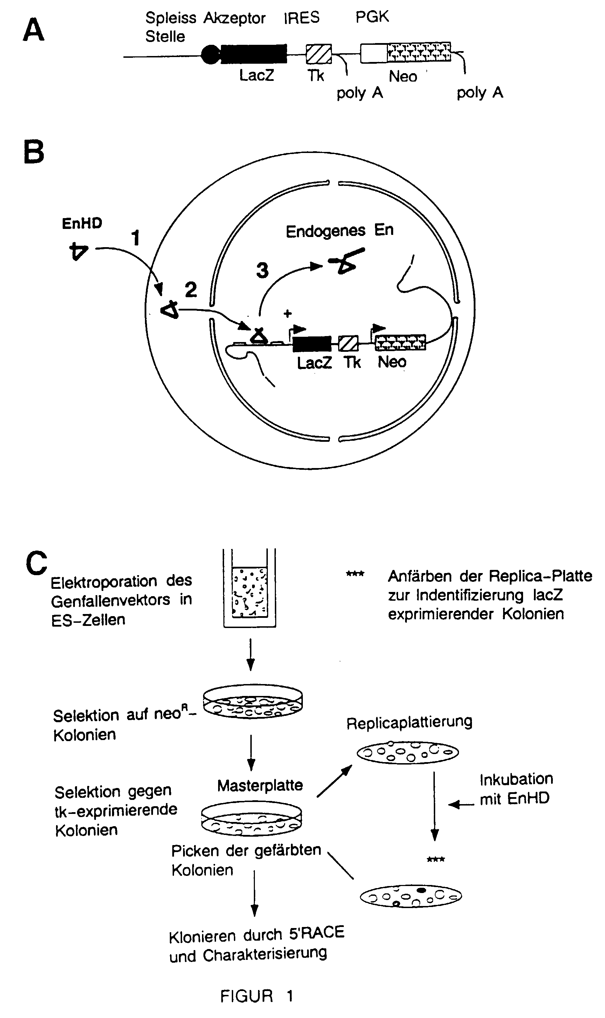

Die beiliegenden Figuren dienen zur weiteren Veranschaulichung der Erfindung. Die Figuren zeigen
Figur 1: Darstellung der Klonierungsstrategie.
   (A) PT2-Vektor mit seiner Spleißakzeptorstelle vor LacZ, der internen Ribosomen-Eintrittsstelle (IRES) vor der Thymidinkinase-(Tk)-cDNA, was die negative Selektion in Gancyclovir und die positive Selektion des NeoR-(Neo)-Gens unter der Kontrolle des Phosphoglyceratkinase-(PGK)-Promotors erlaubt.
   (B) In diesem Schema transloziert die Homöodomäne von Engrailed (EnHD) über die Zellmembran (Schritt 1) und wird in den Nukleus gelenkt (Schritt 2), wo es an verfügbare Stellen bindet und mit endogenen Faktoren, in diesem Falle Engrailed (En), konkurriert. Man nimmt an, daß das Verfahren die Spezifität der Wechselwirkung begünstigt, da die Chromatinstruktur minimal gestört wird.
   (C) 30000 ES-Zellklone wurden nach Elektroporation von linearisiertem PT2-Vektor und Doppelselektion etabliert. Die resistenten Kolonien wurden auf Polyestermembranen replikaplattiert. EnHD wird zu dem Kulturmedium, das die Membranen enthält, gegeben, auf β-gal-Aktivität gefärbt, und die blauen Kolonien werden von der Hauptplatte gepickt. Nach Bestätigung der Induktion in drei unabhängigen Analysen werden die positiven Klone amplifiziert, und die mutmaßlichen Ziele werden mittels 5'-RACE kloniert. Man fand, daß von 30000 Klonen 20 in dem Polyester-Replika-Assay reagierten und 8 wurden in den drei nachfolgenden unabhängigen Induktionen in Mikrotiterlöchern bestätigt.

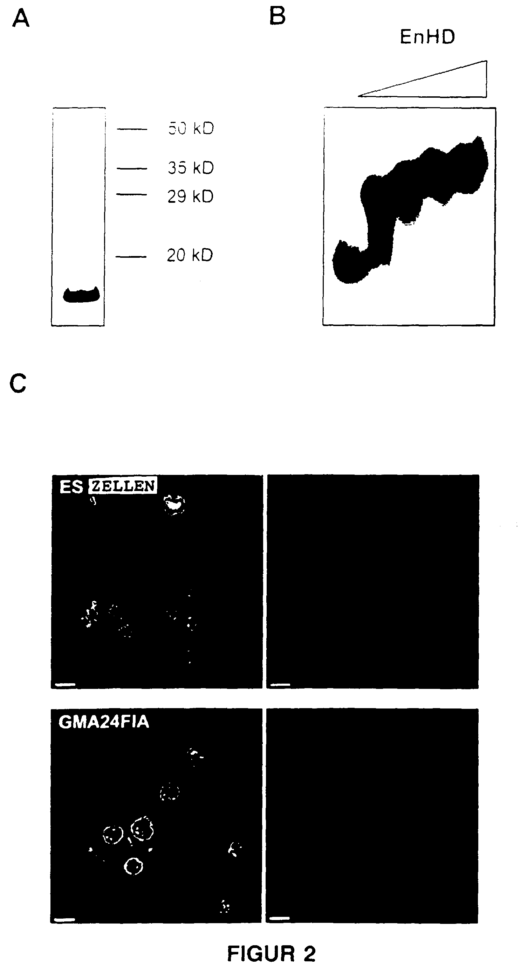

Figur 2: Bakteriell erzeugtes EnHD bindet DNA und wird effizient in Zellen in Kultur internalisiert.
   (A) Polyacrylamid-Gelelektrophorese von aus E. coli-Extrakten gereinigtem EnHD (Coomassie-Färbung).
   (B) Banden-Shift-Assay, durch Inkubieren von 0,5 ng ³²P-ATP-markierten NP6-Oligonukleotiden mit steigenden Mengen (0, 10, 20, 50 und 100 ng) an gereinigtem EnHD.
   (C) ES-Zellen (obere Reihen) oder GMA24FIA (untere Reihen) wurden 2 Std. mit FITC-EnHD (300 ng/ml) (links) oder FITC allein (rechts) inkubiert. Konfokale Schnitte zeigen, daß FITC-EnHD internalisiert wird und in den Nuklei akkumuliert. Maßstab = 5 µm.

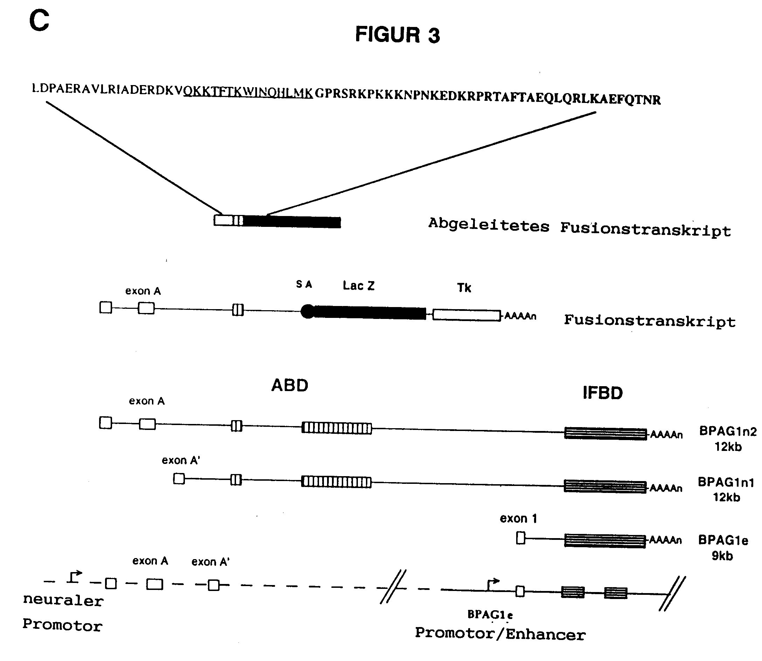

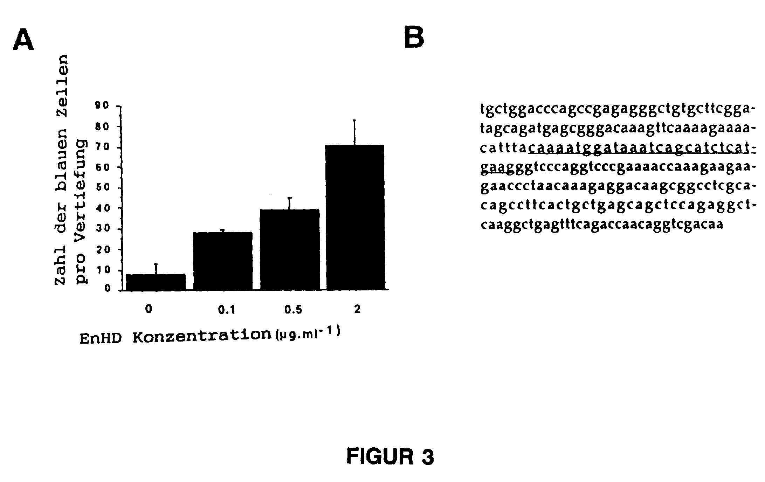

Figur 3: Identifizierung des gefangenen Gens als *dt/BPAG1*-Locus
   (A) 10000 ES-C3-Zellen pro Loch wurden mit steigenden Konzentrationen an EnHD inkubiert. Nach 24 Std. Inkubation wurden die C3-Zellen fixiert und auf β-Galaktosidase-Aktivität gefärbt. Jede Säule veranschaulicht den Durchschnitt von 4 unabhängigen Löchern.
   (B) 5'-RACE von ES-C3-mRNA ergab die Klonierung von 225 Nukleotiden einer BPAG1n2-EnLacZ-Hybrid-cDNA: 56 Nukleotide des Exons A, gefolgt von 45 Nukleotiden der Aktin-Bindungsdomäne (ABD, unterstrichen) und fusioniert an die Spleißakzeptorstelle des Vektors (fett).
   (C) Der *dt/BPAG1*-Locus codiert drei von (mindestens) zwei Promotoren gesteuerte Transkripte. Zwei neurale spezifische Isoformen, BPAG1n1 und BPAG1n2, besitzen eine ABD (schraffierter Kasten) und eine Intermediärfilament-Bindungsdomäne (IFBD) (horizontale Balken). Die BPAG1e-Isoform wird durch einen internen Promotor gesteuert und hat keine ABD-Domäne. Der genomische Bereich, der die ABD codiert, umfaßt mindestens 100 kb, und seine Exon/Intron-Organisation ist noch weitgehend unbekannt. Aus der Sequenz des Hybrid-Transkriptes leiteten wir die Struktur des Fusionsproteins ab: Hinter dem BPAG1n2-spezifischen C-Terminus befinden sich 15 Aminosäuren der ABD (unterstrichen) im Leseraster mit einer EnLacZ-Sequenz (fett). Man nimmt an, daß sich die Integrationsstelle im ersten Intron der ABD befindet.

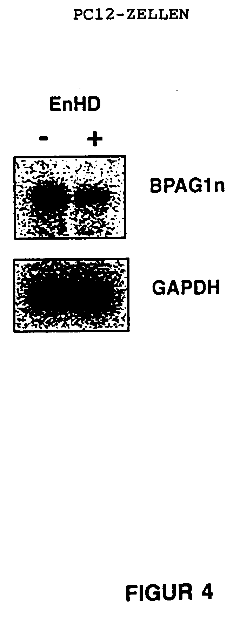

Figur 4: EnHD reguliert *BPAG1n*-mRNA-Spiegel in PC12-Zellen
   (A) Nach drei Tagen Priming mit NGF (50 ng/ml) wurden die PC12-Zellen über Nacht mit EnHD (300 ng/10⁵ Zellen) in Gegenwart von Cycloheximid inkubiert. Die *BPAG1n*-Transkripte wurden durch Northern-Blot-Analyse mit einer Aktin-Bindungsdomänen-Sonde identifiziert. Ein 12 kb-Transkript wurde nachgewiesen, dessen Expression durch Zugabe von EnHD verringert wurde, wohingegen die GAPDH-Expression konstant blieb. Das Experiment wurde dreimal wiederholt, und die Abnahme der mRNA-Anreicherung betrug 30% mit einer Standardabweichung von 7% (Students t-Test, p<0,05).

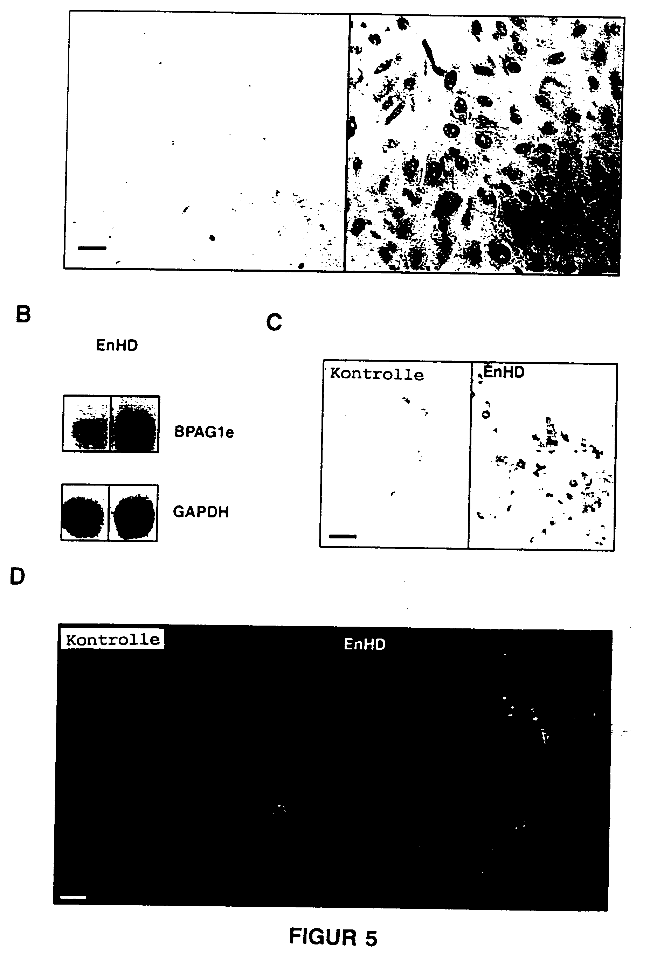

Figur 5: EnHD reguliert *BPAG1e*-mRNA- und BPAG1e-Proteinspiegel in Engrailed-exprimierenden Keratinocyten hoch.
   (A) GMA24FIA-Keratinocyten in Kultur werden mit einem polyklonalen anti-Engrailed(aEnhb-1)-Antikörper gefärbt (rechte Reihe ohne Erstantikörper), Maßstab =
   (B) GMA24FIA-Zellen, über Nacht mit EnHD behandelt (rechte Spur), zeigen einen fünffachen Anstieg der *BPAG1e*-Transkripte, wohingegen die Akkumulation der GAPDH-mRNA nicht modifiziert wird.
   (C) GMA24FIA-Zellen wurden über Nacht mit EnHD in Gegenwart von Cycloheximid inkubiert, und *BPAG1e*-Transkript wurde durch in situ-Hybridisierung nachgewiesen. Maßstab =
   (D) Die Zellen wurden zuerst über Nacht mit Cycloheximid mit oder ohne EnHD behandelt, und das Cycloheximid wurde zwei Stunden vor der BPAG1e-Immunfärbung (BP-Patienten-Autoantiseren, Dr. C. Prost, Paris) entfernt.

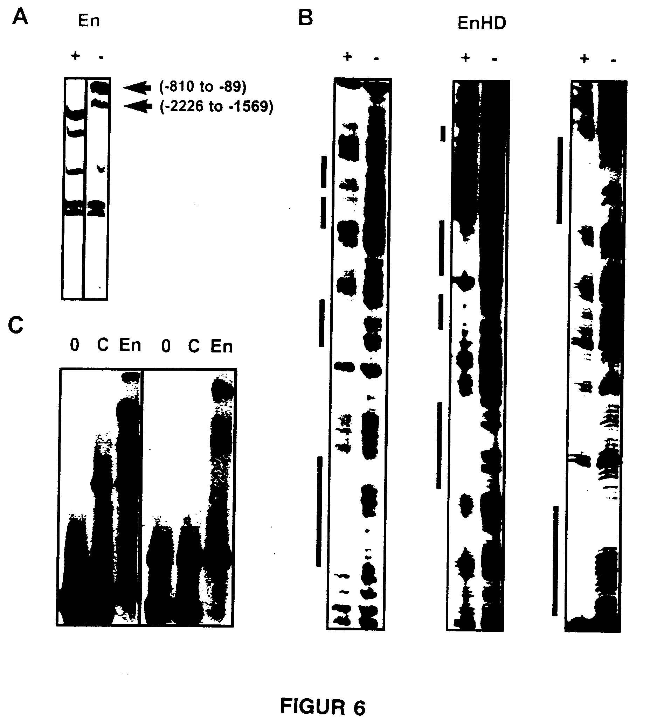

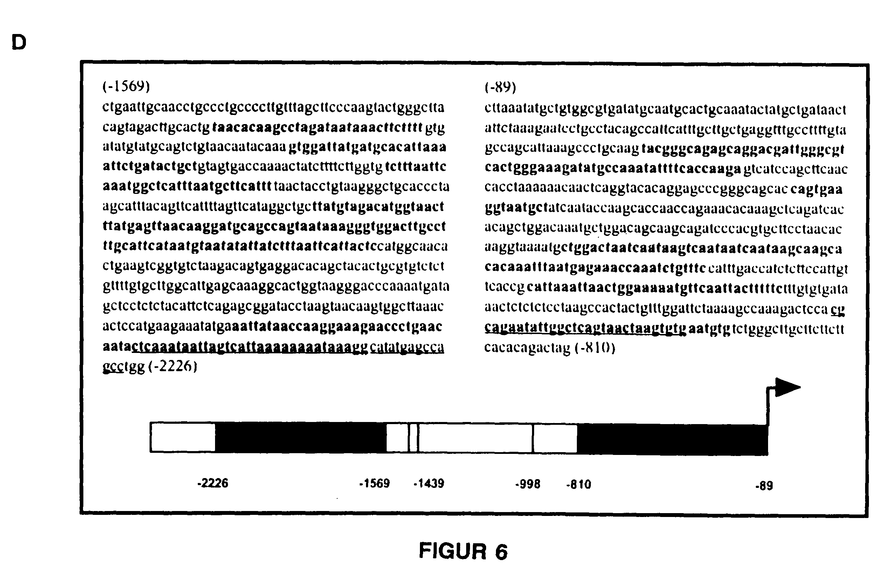

Figur 6: Engrailed bindet an mehrere Stellen im *BPAG1e*-Promotor in vitro.
   (A) Mobilitäts-Shift-Assay von in E.coli produzierten Engrailed-Proteinen an Fragmenten des internen Promotors/Enhancers. Der *BPAG1e*-Promotor wurde mit BstNI gespalten, und die endmarkierten Fragmente wurden auf Bindung an En2 untersucht. Spur 1: Kontrolle mit 2 µl E. coli-Extrakt. Spur 2: 2 µl En2, semigereinigt aus einem E. coli-Extrakt. Zwei Fragmente (-810 bis -89 und 2226 bis -1569, Minus-Strang) zeigen eine hochaffine Bindungsaktivität.
   (B) DNase I-Footprint-Analyse der Promotorfragmente mit gereinigtem EnHD. Die Fragmente wurden endmarkiert und mit EnHD wie in den experimentellen Verfahren beschrieben inkubiert. Die beiden Spuren linksaußen entsprechen dem Fragment -2226 bis - 1569. Die folgenden vier Spuren entsprechen dem Fragment -810 bis -89 mit einem langen Lauf (Mitte) und einem kurzen Lauf (rechts). Die Footprints sind durch schwarze Balken angedeutet. 0 ist die Kontrolle ohne Protein und 10 ist EnHD (10 µl, 6 ng).
   (C) Gelshift-Analyse von zwei im Footprint untersuchten Bereichen (in 6D unterstrichen) mit Vollängen-En2. Die Gelshifts in der rechten Reihe wurden mit dem Oligonukleotid ctcaaataattagtcattaaaaaaaataaaggcatatgagccagcct und in der linken Reihe mit dem Oligonukleotid cgcagaatattggctcagtaactaagtgtg durchgeführt. 0, kein Extrakt; C, E. coli-Extrakt; En, En2-exprimierender E. coli-Extrakt.
   (D) Sequenz der beiden im Footprint untersuchten Bereiche (schwarze Kästen) des BPAG1e-Promotors. Die geschützten Bereiche sind fett markiert.

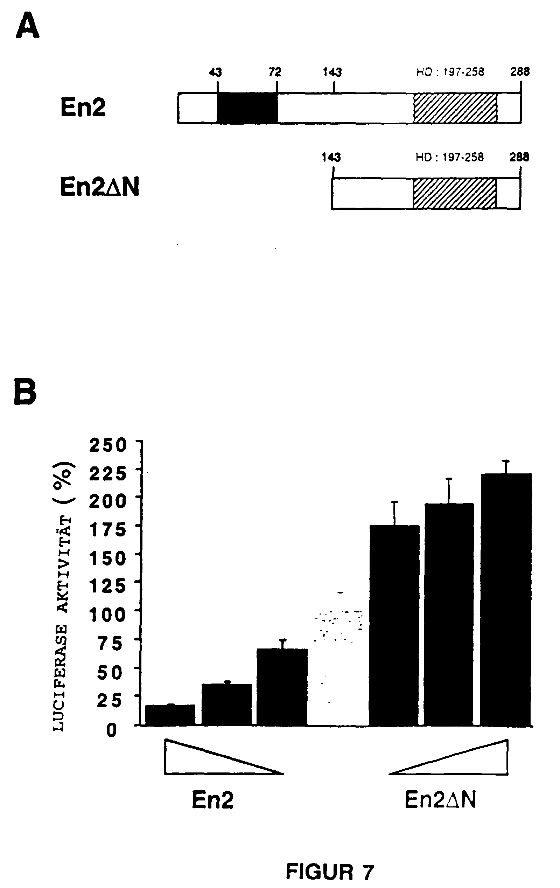

Figur 7: Engrailed2 reprimiert und EnHD aktiviert die *BPAG1e*-Promotor-Aktivität.
   (A) Schematische Darstellung der beiden zur Cotransfizierung mit pBP-luc verwendeten Konstrukte.
   (B) GMA24FIA-Zellen wurden mit pBP-luc allein oder mit steigenden En2- oder En2ÆN-Dosen wie angegeben transfiziert. Nach 48 Stunden wurde die Luciferase-Aktivität in Zellysaten gemessen. Jeder Wert entspricht dem Durchschnitt von 4 unabhängigen Löchern. Die Wirkungen der beiden Konstruktionen sind entgegengesetzt und dosisabhängig.

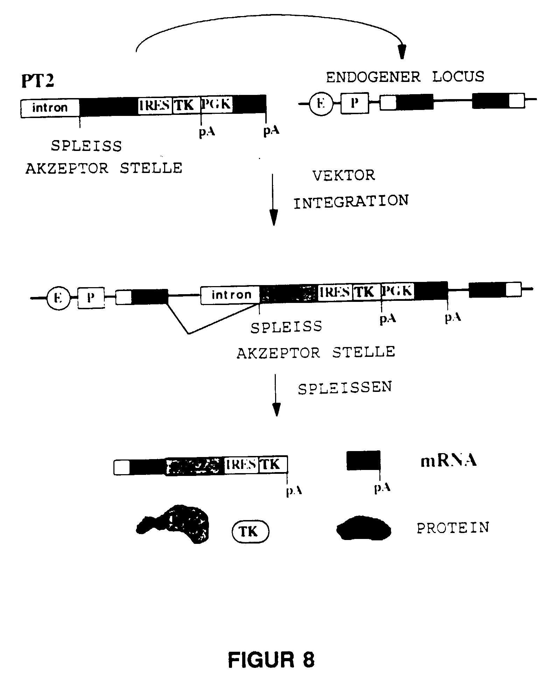

Figur 8: Darstellung des PT2-Vektors und seiner Funktion im erfindungsgemäßen Verfahren.

### Experimentelle Strategie

Die Figur 1 veranschaulicht das Prinzip des erfindungsgemäß entwickelten neuen Verfahrens. ES-Zellen werden mit einem linearisierten PT2-Genfallenvektor elektroporiert. Dieser Vektor enthält ein Reportergen *lacZ*, das im Leseraster an eine Spleißakzeptorstelle fusioniert ist, gefolgt von einem *Thymidinkinase-Gen (tk)*, das an eine interne Ribosomen-Eintrittsstelle (Internal Ribosomal Entry Site, IRES) gebunden ist. Somit wird eine bicistronische Botschaft hergestellt, die lacZ und tk enthält (Figur 1A). Durch Zugabe von Gancyclovir zu dem Medium können Klone, die das *tk*-Gen exprimieren, eliminiert werden. Dadurch wird gegen Integrationen in konstitutiv aktive Gene selektiert. Ein zweites Gen, das eine Neomycinresistenz kodiert und unter der Kontrolle des *Phosphoglyceratkinase-(PGK)*-Promotors steht, ermöglicht eine positive Selektion transfizierter Klone. Die Klone werden dann auf Polyesterfilter replikaplattiert und mit EnHD inkubiert. Unter der Annahme, daß Engrailed die Transkription hauptsächlich über eine N-terminale Domäne unterdrückt, die in EnHD fehlt, kann die Einbringung von EnHD durch Konkurrenz mit endogenem Engrailed die Repression aufheben (Figur 1B). Dadurch können *LacZ*-exprimierende Klone auf den Polyesterfiltern und danach auf der Hauptplatte identifiziert werden. Die mutmaßlichen Engrailed-Ziele können dann mit 5'-RACE-PCR-Strategien (Figur 1C) kloniert werden. In diesem Schema (Figur 1B) ist das Beispiel aufgeführt, in dem *LacZ* in einem direkten Engrailed-Ziel integriert ist. *LacZ* kann aber auch nach Integration in ein Gen exprimiert werden, das indirekt durch Engrailed reguliert wird (bspw. in seinem genetischen Regulationsweg.

In dieser Studie verwendeten wir die Engrailed-Homöodomäne (EnHD) aus Hühnchen, da spezifische Antikörper zur Verfügung stehen, die zwischen dem Vogel-Polypeptid und dem endogenen Säugetier-Engrailed unterscheiden können. Die hohe Sequenzkonservierung zwischen den unterschiedlichen EnHDs und die normale Entwicklung einer Maus, in der *En1* mutiert wurde und durch *En2* ersetzt wurde, ergab, daß die bei dieser Studie verwendete Homöodomäne im Erfolgsfalle die Identifizierung von En1- und En2-Zielen in Säugetieren ermöglicht.

### Die Engrailed-Homöodomäne wird durch Zellen in Kultur internalisiert.

Das in Figur 1 beschriebene Protokoll erfordert, daß das zu dem Kulturmedium zugegebene EnHD von einer großen Anzahl ES-Zellen aufgenommen wird. EnHD wurde in *E. coli* produziert und auf Heparin-Sepharose gereinigt. Die Reinheit von EnHD war, bestimmt durch SDS-PAGE-Analyse und Färbung mit Coomassie-Blau, größer als 80% (Figur 2A). Zur Untersuchung der EnHD-Bindungseigenschaften wurden Gelshift-Experimente durchgeführt, bei denen ein DNA-Fragment mit 6 Kopien der NP6-Stelle (TCAATTAAAT) verwendet wurde, die bekanntlich an Drosophila-Engrailed bindet. Wie aus Figur 2B ersichtlich ist, entstehen 4 bestimmte Komplexe, wenn die EnHD-Menge schrittweise von 10 ng auf 100 ng erhöht wird. Dies zeigt, daß EnHD in vitro effizient an NP6 bindet.

EnHD wurde mit Fluorescein-Isothiocyanat (FITC) wie schon beschrieben markiert, und zu ES-Zellen in Kultur für 2 Std. bei 4°C gegeben. Die Figur 2C (obere Reihen) zeigt, daß EnHD von den ES-Zellen effizient aufgenommen wird. Keine Färbung wurde mit nur FITC (obere und rechte Reihe) beobachtet, und konfokale Schnitte bestätigten, daß das Peptid tatsächlich im Inneren der Zelle vorliegt. Ähnliche Ergebnisse wurden mit FITC-markiertem EnHD und durch Immunocytochemie (nicht gezeigt) in GMA24FIA-Keratinocyten (Figur 2C, untere Reihen), einer in dieser Studie verwendeten Zellinie (siehe unten), erhalten. Zusammengefaßt folgern wir, daß EnHD durch Zellen in Kultur effizient eingefangen wird.

### Identifizierung eines mutmaßlichen Engrailed-Zielgens.

Eine Bank aus 30000 unabhängigen Genfallenvektor-Integrationen in ES-Zellen wurde auf die Reaktion auf Engrailed durchmustert, indem EnHD zu dem Kulturmedium nach dem in Figur 1 schematisch dargestellten Protokoll gegeben wurde. Dadurch konnten 20 reagierende Klone identifiziert werden. Die Reaktion dieser 20 Klone wurde anschließend in 96-Loch-Platten in drei unabhängigen Experimenten wiederholt. Acht Klone zeigten durchweg eine Induktion der Reportergenexpression. Die Figur 3A zeigt für den Klon ES-C3, dem Gegenstand des vorliegenden Experiments, wie die Anzahl der *LacZ*-exprimierenden Zellen auf eine dosisabhängige Weise durch Zugabe von EnHD erhöht wird.

Das von der Genfallenvektorintegration in Klon ES-C3 erzeugte Fusionstranskript wurde durch 5'-RACE charakterisiert. Ein etwa 225 bp großes Fragment wurde amplifiziert, kloniert und danach sequenziert. Diese Sequenz (Figur 3B) weist eine Hybrid-cDNA auf, die den Spleißakzeptor der Genfallenvektorsequenz und 101 Nukleotide enthält, die zu 100% mit einer Sequenz identisch sind, die in der neuralen Isoform vom Typ II von *Dystonin* (Genebank, Bez. gb U25158), auch als *BPAG1n2* (*bullöses Pemphigoid Antigen neural 2*) bezeichnet, gefunden wird. In dieser Sequenz entsprechen 45 Nukleotide dem 5'-Ende des Exons, das den C-Terminus der Aktin-Bindungsdomäne von Dystonin entspricht, und 56 Nukleotide dem *Dystonin*-Exon A (Figur 3C).

Die *BPAG1n2*- und *LacZ*-Sequenzen liegen im Raster, was die Expression eines Fusionsproteins ermöglicht und mit der beobachteten β-Galaktosidase-Aktivität übereinstimmt. Die Existenz dieser Hybrid-cDNA wurde durch RT-PCR bestätigt, wobei zwei unabhängige PCR-Primersätze verwendet wurden. Um weiter zu zeigen, daß die Hybrid-mRNA in ES-C3-Zellen vorhanden war, wurde ein RNase-Protektions-Test durchgeführt, bei dem eine Antisense-RNA-Sonde aus der *BPAG1n2-LacZ*-cDNA und Gesamt-RNA aus ES-C3-Zellen und normalen ES-Zellen verwendet wurden. Erwartungsgemäß wurde ein 200 Nukleotide großes Fragment, das der Hybrid-mRNA entspricht, in der RNA aus ES-C3-Zellen geschützt, jedoch nicht in der Kontroll-RNA (Daten nicht gezeigt).

Die Organisation des *dt/BPAG1*-Locus ist in Figur 3C beschrieben. Der *dt/BPAG1*-Locus codiert mindestens drei unterschiedliche Isoformen, die BPAG1n1, BPAG1n2 und BPAG1e codieren. Die beiden ersteren Isoformen werden im Nervensystem und die letztere in Keratinocyten der epidermalen Basalschicht exprimiert. Der Aminoterminus der neuralen Isoformen unterscheidet sich von dem von BPAG1e durch die Anwesenheit einer funktionellen Aktin-Bindungsdomäne (ABD), wohingegen der bei allen drei Isoformen gleiche Carboxyterminus eine Intermediärfilament-Bindungsdomäne (IFBD) enthält. Anhand der Hybrid-cDNA-Sequenz läßt sich ersehen, daß der Genfallenvektor am wahrscheinlichsten in ein Intron der ABD-Region des *dt/BPAG1*-Locus integrierte, was zur Verkürzung der beiden neuralen Isoformen führte (Figur 3C).

### Ex vivo-Regulation der BPAG1n-Expression durch EnHD.

Zur weiteren Untersuchung der genetischen Regulation zwischen Engrailed und *Dt/BPAG1n* verwendeten wir neuronale Zellen, die beide Gene exprimieren. Die von Phaeochromocytom abstammenden PC12-Zellen aus Ratte nehmen einen Phänotyp an, der einem sympathischen Neuron ähnelt, und exprimieren *Dt/BPAG1n* und *En1* nach Zugabe von Nervenwachstumsfaktor (NGF). Da die Homöodomänen von En1 und En2 funktionell identisch sind, sollte EnHD an En1-Zielstellen binden und somit mit endogenem Engrailed konkurrieren können. Die PC12-Zellen wurden drei Tage mit NGF inkubiert und über Nacht mit EnHD (300 ng/10⁵ Zellen) in Gegenwart von Cycloheximid behandelt. Die Figur 4A zeigt eine Northern-Blot-Analyse, durchgeführt mit einer *dt-ABD*-Sonde, die die beiden, den beiden neuralen Isoformen entsprechenden mRNAs erkennt. Die beiden mRNAs können nicht aufgrund ihrer Größe unterschieden werden, und man wies eine einzelne Bande von 15 kb mit einer zweifachen Abnahme der mRNA-Anreicherung nur 12 Stunden nach Zugabe von EnHD nach. Diese mittels Phosphoimager-Analyse quantifizierte Abnahme beruht nicht auf einer generellen Abnahme der mRNA-Transkription oder Anreicherung, da die Expression von *Glycerinaldehyd-3-phosphat-Dehydrogenase (GAPDH)* unverändert blieb. Das gleiche Experiment wurde dreimal wiederholt und ergab qualitativ identische Ergebnisse (Legende in Figur 4). Da Cycloheximid die Translation hemmt, deuten die Ergebnisse der letzteren Experimente darauf hin, daß EnHD in diesem Modell direkt auf die Regulation der *BPAG1n*-Transkription wirkt.

### Ex vivo-Regulation der BPAG1e-Expression durch EnHD.

Zusätzlich zu den überlappenden Expressionsmustern von *BPAG1n* und *Engrailed* im ZNS, beobachteten wir, daß En1 und BPAG1e auch in Keratinocyten der epidermalen Basalschicht coexprimiert werden (nicht gezeigt). Wir untersuchten daher, ob die epidermale Isoform (*BPAG1e*) neben den neuronalen BPAG1-Isoformen auch durch Engrailed reguliert wurde. Zu diesem Zweck untersuchten wir die Expression von BPAG1e und Engrailed in einer immortalisierten Keratinocyten-Zellinie aus Mensch (GMA24FIA-Zellinie), die sich wie basale Keratinocyten verhalten. αEnhb1, ein polyklonaler Antikörper, der En1 und En2 erkennt, färbt die Zellnuklei (Figur 5A) und erkennt im Western-Blot (nicht gezeigt) ein einzelnes 52 kD-Protein, was zeigt, daß diese Zellen En1 exprimieren. Mit Hilfe einer Northern-Blot-Analyse wurde ein einzelnes Transkript von 9 kb, das der *BPAG1e*-mRNA entspricht (Figur 5B), jedoch keine 15 kb-Transkripte, die den *BPAG1n*-Isoformen entsprechen, nachgewiesen. Wir bewiesen, daß GMA24FIA-Zellen EnHD internalisieren (Figur 2D) und analysierten dann die Auswirkung der Homöodomänen-Internalisierung auf die *BPAG1e*-Expression. Wie in Figur 5B gezeigt verstärkt die Zugabe von EnHD über Nacht die Menge an *BPAG1e*-mRNA um mindestens einen Faktor 5. Die in situ-Hybridisierungen von Figur 5C zeigen sogar in Gegenwart von Cycloheximid eine starke Wirkung von EnHD auf die Menge der BPAG1e-mRNA, was darauf hinweist, daß die Regulation direkt erfolgt. Schließlich wurden die GMA2-4FIA-Zellen über Nacht mit oder ohne EnHD in Gegenwart von Cycloheximid inkubiert, gewaschen und weitere 2 Std. ohne Cycloheximid inkubiert, was eine de novo-Proteinsynthese ermöglichte.

Wie in Figur 5D veranschaulicht ist, hatte EnHD eine starke positive Wirkung auf die BPAG1e-Synthese und die Anreicherung unter der Zellmembran. Diese Daten zeigen, daß die *BPAG1e*-Expression durch EnHD in GMA24FIA-Zellen aktiviert wird.

### Ein interner Promotor/Enhancer von BPAG1 ist ein Ziel des Engrailed-Proteins.

Innerhalb des *BPAG1*-Locus wurde ein interner Promotor/Enhancer identifiziert (s. Figur 6). Dieser steuert die in vivo-Expression eines *lacZ*-Reportergens in epidermalen Keratinocyten, sowie in Bereichen des ZNS, die Engrailed exprimieren (z.B. Mittelhirn). Er kann somit als Regulator der beiden *BPA*-*G1n*-Isoformen und der *BPAG1e*-Isoform wirken. Zur Lokalisierung der mutmaßlichen Bindungsstellen für Engrailed in dem internen *BPAG1*-Promotor/Enhancer wurden Gelshift-Assays durchgeführt, bei denen markierte BstN1-Restriktionsendonuklease-Fragmente der (-2466 bis -93) Promotor-Region und partiell gereinigtes En2 (En2)-Protein aus Hühnchen verwendet wurden. Wie Figur 6 A zeigt, verschieben sich die beiden größeren Fragmente (-2226 bis -1569) und (-810 bis -89) in Gegenwart von En2 (Pfeile), was die Existenz von funktionellen Bindungsstellen anzeigt.

Die beiden Fragmente wurden mittels DNase I-Footprinting mit EnHD analysiert. Die Footprintreaktionen erfolgten mit beiden markierten Strängen und ergaben identische Ergebnisse. Es sind daher für jedes Fragment nur die Ergebnisse für jeweils einen Strang gezeigt Die Fragmente (-810 bis -89) und (-2226 bis -1569) zeigten jeweils 5 spezifische Footprints (Figur 6B). Wie Figur 6D zeigt, enthalten einige der geschützten Bereiche (fett) die ATTA-Konsensuskernsequenz für Homödomänenproteine. Unter diesen sind einige fast identisch mit der NP6-Sequenz TCAATTAAAT, die nachgewiesenermaßen ein Ziel für Engrailed in Drosophila ist. Zwei EnHD-Footprints enthalten keinerlei ATTA-Sequenzen (Figur 6D).

Um zu testen, ob diese EnHD-Bindungsstellen auch durch Vollängen-Engrailed 2 erkannt werden, wurden Gelshift-Assays mit bakteriell erzeugtem En2 durchgeführt. Zwei den beiden unterschiedlichen geschützten Bereichen entsprechende Oligonukleotidpaare wurden verwendet. Eines (-2225 bis - 2180) enthält drei bona fide ATTA-Motive, das andere (-694 bis 665) hat keinerlei ATTA-Sequenz (in Figur 6D unterstrichen). Wie Figur 6C zeigt, bildete En2 zwei sich verlagernde Komplexe mit beiden Oligonukleotidpaaren, was zeigt, daß En2 effizient mindestens zwei durch EnHD in der Footprint-Analyse geschützte Sequenzen bindet, einschließlich einer Sequenz ohne ATTA-Kern.

### Die Aktivität des internen BPAG1-Promotors/Enhancers wird durch En2 reguliert.

Das genomische 2,3 kb HindIII/BamHI-Fragment (Position - 2463 bis -89) von *BPAG1e*, durch das Reportergene, die in Keratinocyten und im ZNS exprimiert werden, spezifisch exprimiert werden, wurde in einem von pGL2 abgeleiteten Vektor (pBP-luc) vor das Luciferasegen plaziert. Das Reportergen wurde in GMA24-FIA-Zellen mit einem Plasmid, das das Vollängen-En2 codiert oder einem Plasmid, das En2 mit deletierter N-terminaler Domäne (En2ÆN) codiert, cotransfiziert. Dem letzteren, En2ÆN, fehlt die Domäne, die für die Vermittlung aktiver Repression verantwortlich ist. Man erwartet daher, daß es wie EnHD wirkt. Das cotransfizierende pBP-luc mit steigenden pEn2ÆN-Mengen ergibt einen dosisabhängigen Anstieg der Promotoraktivität, wohingegen das Vollängen-En2 die gegenteilige, jedoch auch dosisabhängige Wirkung zeigt (Figur 7). Die Cotransfektion steigender Mengen beider Plasmide mit einem β-Galaktosidase exprimierenden Plasmid zeigte, daß keines der beiden Plasmide einen Einfluß auf die Zellüberlebensfähigkeit hatte (nicht gezeigt). Zusammengefaßt schließen wir, daß En2 die BPAG1e-Promotoraktivität direkt reguliert und daß in GMA24FIA-Zellen En2 und En2ÆN entgegengesetzt auf diese Aktivität wirken.
Die Genfallentechnologie wurde hier zur Identifizierung und zur zufallsmäßigen Mutation von Genen in ES-Zellen verwendet. Diese Genfallenintegrationen können weiter auf spezifische Expressionsmuster in vivo und in vitro durchmustert werden. Wir haben diese Technologie zur Identifizierung und Mutation von Genen, die durch homöodomänenhaltige Proteine reguliert werden, weiter ausgearbeitet. Dieser neue Ansatz hat gegenüber anderen Technologien, die auf die Identifizierung differentiell exprimierter Gene zielen, bspw. dem Zwei-Hybrid-System und dem Differentialdisplay, mehrere Vorteile. Die markierten Zielgene können mit PCR-Strategien leicht kloniert und charakterisiert werden. Die Expression der eingefangenen Zielgene kann untersucht werden, indem man die Reportergenaktivität in Chimären und F1-Nachkommen verfolgt, so daß sich zell- und gewebespezifische Wechselwirkungen vorherbestimmen lassen. Die Reportergenexpression kann als nützlicher Marker verwendet werden, mit dem das Netzwerk genetischer Wechselwirkungen analysiert wird. Die Integrationen können einen mutierten Phänotyp hervorrufen, der nach der Keimbahntransmission untersucht werden kann.

Zur Identifizierung seltener Genfallen-Vektor-Integrationen in Gene, die in spezifischen genetischen Reaktionswegen stromabwärts aktiv sind, wurde das klassische Durchmusterungsverfahren verbessert. Zu diesem Zweck wurde ein *Thymidinkinase*-Gen, fusioniert an eine interne Ribosomen-Eintrittsstelle (IRES) 3' vom *LacZ*-Gen, in dem PT1-Genfallenvektor eingeschlossen. Die meisten Integrationen in konstitutiv aktive Gene konnten somit durch die Zugabe von Gancyclovir in das Selektionsmedium eliminiert werden. Die Verwendung dieses neuen bicistronischen Vektors, in Kombination mit der Replikaplattierungstechnik, ermöglichte die Durchmusterung einer großen Zahl von Genfallenvektor-Integrationen und die spezifische Identifizierung von Zielgenen exogen zugegebener homöodomänenhaltiger Transkriptionsfaktoren.

### Identifizierung von Engrailed-Zielgenen durch Translokation.

Die Homödomäne von Antennapedia (AntpHD), Hoxa5HD und FtzHD sowie Vollängen-Hoxa-5 und Hoxc-8 kann in das Cytoplasma und den Nukleus lebender Zellen in Kultur eindringen. Obwohl der Translokationsmechanismus über die biologische Membrane nicht vollständig verstanden ist, deuten Experimente mit mehreren Peptidvarianten, abgeleitet von der dritten AntpHD-Helix, daraufhin, daß die Translokation keinen chiralen Rezeptor erfordert und keine klassische Endocytose beinhaltet. Ein klarer Vorteil dieser Technik ist, daß die Polypeptide in das Cytoplasma und den Nukleus gelangen können, ohne daß sie durch den Endocytoseweg abgebaut werden und sich in einer ungestörten Umgebung befinden. Dieser letzte Punkt ist besonders bei der Einführung von Vollängen-Homöodomänen oder -Homöoproteinen wichtig, die, wenn sie sich in den Nuklei befinden, nur an Stellen binden, die durch die Chromatinstruktur zugänglich gemacht wurden. Daher wird die Menge an nichtspezifischen Interaktionen verringert, wenn nicht sogar eliminiert.

### Regulierung der BPAG1n-Expression durch Engrailed.

In dieser Studie identifizierten wir 8 unabhängige Genfallenvektorintegrationen in ES-Zellen, die auf zu dem Kulturmedium zugegebenes EnHD reagieren. Bei einem dieser Klone, ES-C3, erfolgte die Integration im *dt/BPAG1*-Locus. Die Integrationsstelle des Genfallenvektors in einem Intron der ABD-Domäne von BPAG1n wies darauf hin, daß *BPAG1n* stromabwärts von Engrailed liegt. Die in situ-Hybridisierung zeigt, daß *BPAG1n* in mehreren Domänen des embryonalen ZNS der Maus exprimiert wird, einschließlich Bereichen, in denen *Engrailed* nicht exprimiert wird. Die Expression außerhalb von *Engrailed*-Domänen weist darauf hin, daß *BPAG1n* auch ein Ziel für andere regulatorische Proteine, möglicherweise, jedoch vermutlich nicht ausschließlich andere Homöproteine, ist. Da die Erkennungsstellen für die Bindung von Homöoproteinen ubiquitär sind, erwartet man, daß von verschiedenen Homöoproteinen und Homöodomänen ähnliche oder sogar identische Promotorregionen erkannt werden.

Die in vivo-Abnahme der BPAG1n-Expression in *Engrailed*-Mutanten bestätigt daher, daß BPAG1n sich in dem genetischen Regulationsweg von *Engrailed* befindet. Die letzte Beobachtung scheint zu zeigen, daß sich Engrailed in E12.5-Embryos wie ein Aktivator der *BPAG1n*-Expression verhält, wohingegen Engrailed in ES-Zellen, in Keratinocyten und im Dermomyotom wie ein Repressor wirkt. Dies stimmt mit der Funktion von *Drosophila*-*Engrailed* überein, das je nach der Entwicklungsstufe und dem betrachteten zellulären Zusammenhang ein Repressor oder Aktivator sein kann. Es ist in diesem Zusammenhang bemerkenswert, daß die Aktivität des LacZ-Reportergens, gesteuert vom internen *BPAG1-*Promotor/Enhancer im Mesencephalon von E8.5- bis zu E12.5-Mäusen, durch positive Regulation eines Engrailed-responsiven Elementes in einem neuronalen Engrailed-exprimierenden Gebiet verstärkt wird.

### Regulation der BPAG1e-Expression durch Engrailed.

Der *dt/BPAG1*-Locus codiert auch BPAG1e, ein Protein, das in Keratinocyten Keratinfilamente mit Hemidesmosomen verbindet, und somit bei der Aufrechterhaltung der Hemidesmosomenstruktur wichtig ist. Die interne Promotor/Enhancer-Sequenz stromaufwärts von *BPAG1e* enthält einige mutmaßliche Homöoprotein-Bindungsstellen. Neben den in situ-Hybridisierungsstudien, die zeigen, daß das Ausmaß der *BPAG1e*-Expression in der Epidermis bei Fehlen von *Engrailed* beträchtlich erhöht war, deutete die letztere Beobachtung auf eine genetische Wechselwirkung zwischen *BPAG1e* und *Engrailed* hin. Wir bestätigten daher, daß *En1* und *BPAG1e* in basalen Keratinocyten in vivo (nicht gezeigt) und in der immortalisierten Keratinocyten-Zellinie GMA24FIA exprimiert werden. Mit Hilfe dieser Zellinie konnten wir zeigen, daß die Zugabe von EnHD die *BPAG1e*-Expression steigert, und daß dieser Anstieg auch in Gegenwart von Cycloheximid beobachtet wird. Wie im Fall von BPAG1n, deuten diese Experimente stark auf eine direkte Regulation von *BPAG1e* durch *Engrailed* hin, schließen jedoch nicht vollständig eine Wirkung des EnHD auf die Stabilität der *BPAG1e*-mRNA aus. Es ist ebenfalls bemerkenswert, daß die Zugabe von EnHD die Transkriptmenge erhöht, was zeigt, daß Engrailed in Keratinocyten im Gegensatz zu dem Geschehen in PC12 als Repressor wirkt.

### dt/BPAG1 enthält eine Promotor/Enhancer-Sequenz, die direkt durch Engrailed reguliert wird.

Die Gegenwart eines internen Promotors stromaufwärts von *BPAG1e* war sehr nützlich bei der Bestätigung der Gültigkeit des Ansatzes, der in der vorliegenden Erfindung zur Identifizierung mutmaßlicher Engrailed-Zielgene verwendet wurde. Dieser Promotor ist von besonderem Interesse, da er die Expression von *LacZ* im Mesencephalon steuert, was darauf hinweist, daß entweder *BPAG1e* im ZNS exprimiert wird oder, und nicht ausschließlich, daß diese Domäne im Nervensystem für die *BPAG1n*-Expression als *Engrailed*-Enhancer wirkt. Die letztere Interpretation wird durch die Tatsache unterstützt, daß das durch den internen Promotor gesteuerte *LacZ* ähnlich wie *BPAG1n* in der Engrailed-exprimierenden Domäne hochreguliert wird.

### Allgemeingültigkeit des Ansatzes.

Es konnte in der vorliegenden Erfindung gezeigt werden, daß zellpermeable Peptide zur Identifizierung von beispielsweise Homöoprotein-Zielen verwendet werden könnte. Es ist außerdem offensichtlich, daß das hier vorgestellte Verfahren durch Verknüpfen von Polypeptiden an die dritte AntpHD-Helix und somit durch ihre Internalisierung nun auch auf andere Proteine ausgedehnt werden kann, für die aktive Domänen identifiziert worden sind, bspw. eine andere DNA-bindende Domäne als die Homöodomäne. Bisher gelantgen sämtliche von uns synthetisierten und hergestellten Polypeptide mit Längen unter 100 Aminosäuren in das Cytoplasma der Zellen und in den meisten Fällen in ihren Nukleus. Da die dritte AntpHD-Helix, die als Vektor dienen kann, 16 Aminosäuren umfaßt, können wahrscheinlich aktive Domänen mit einer minimalen Länge von 84 Aminosäuren in lebende Zellen eingebracht werden. Dies eröffnet den Weg zu einer neuen und leistungsfähigen Induktions-Genfallen-Technologie.

Vorstehend wurde die Erfindung anhand eines speziellen Genfallenvektors und des Engrailed 2 Proteins beschrieben. Selbstverständlich ist die Erfindung nicht auf dieses spezielle Ausführungsbeispiel beschränkt. Der Fachmann kann dieses Ausführungsbeispiel auf der Grundlage der vorstehenden Anmeldung abändern, ohne von der beschriebenen Erfindung abzuweichen.

### Experimentelle Verfahren

### Zellkultur

ES-Zellen wurden in dem D3-ES-Zellwachstumsmedium [Wurst und Yoyner, 1993] gezüchtet. Primäre Cerebellum- und Metencephalon-Kulturen aus Rattenhirn (P0) wurden wie zuvor beschrieben hergestellt [Joliot, 1991]. PC12-Zellen wurden in DMEM/F12-Medium, das 10% Pferdeserum (Seromed) und 5% fötales Kälberserum (GIB-CO) enthielt, gezüchtet. Die für die Experimente verwendeten PC12-Zellen wurden drei Tage mit 50 ng/ml NGF (Boehringer) inkubiert. GMA24FIA ist ein Feeder-unabhängiger Subklon des menschlichen Keratinocyten-Stammes GMA [Barrandon, 1989]. GMA24FIA wurden in normalem KGM gezüchtet, das mit EGF angereichert war [Rochat, 1994].

### Genfalle

Das Genfallenverfahren ist genau in [Hill und Wurst, 1993a,b] beschrieben. Hier wird jedoch eine kurze Übersicht gegeben: Die ES-Zellen wurden vermehrt und mit einem HindIII-linearisierten Genfallenvektor PT2 elektroporiert [Wurst, 1993]. Nach der Elektroporation wurden die Zellen auf gelatinebeschichteten 10-mm-Platten ausplattiert und 8 Tage in ES-Medium gezüchtet, das G418 (200 µg/ml) und 2 mM Gancyclovir enthielt. Nach 8tägiger Selektion wurde eine Polyestermembran auf die Zellen gelegt, mit Glasperlen beschichtet, Medium wurde zugegeben, und die Zellen wurden weitere 48 Std. gezüchtet. Dann wurden die Polyesterfilter entfernt, in PBS gespült und in einer neuen Schale mit Induktionsmedium untergebracht, das EnHD (30 ng/10⁴ Zellen) enthielt. Nach 48 Stunden wurden die Membranen fixiert und wie in Hill und Wurst (1993) beschrieben auf β-Galaktosidaseaktivität gefärbt. β-Galaktosidase-positive Klone wurden gepickt, vermehrt und erneut in 96-Loch-Platten in drei unabhängigen Experimenten untersucht.

### Expression und Reinigung von EnHD und Engrailed 2 aus Hühnchen.

Die 60 Aminosäuren große Homöodomäne des Engrailed 2-Proteins (EnHD) aus Hühnchen wurde aus einem Plasmid, das die Hühnchen-cDNA-Sequenz enthielt, mit den folgenden Primern isoliert:
5'-TTTCATATGGAAGACAAGCGGCCCCG-3' und
5'-GGGGGATCCCTACGCCTTCTTGATCTTGGCTC-3'.

Das PCR-Produkt wurde in pBluescript ligiert. Das Konstrukt wurde dann in den pET-3a-Expressionsvektor subkloniert. Das Plasmid wurde in BL21(DE3)pLysS transformiert, und die Expression von EnHD durch Zugabe von 0,8 mM IPTG induziert. Das Protein wurde isoliert, indem die Bakterien in Puffer A (20 mM HEPES, pH-Wert 7,9, 1 mM EDTA, 5 mM MgCl₂, 0,2 M NaCl, 0,02 mg/ml DNase I und Proteaseinhibitor: 0,5 mM Pefablock, 10 µg/ml Pepstatin, 10 µg/ml Aprotinin, 1 µg/ml α2-Makroglobulin) ultraschallbehandelt wurden, gefolgt von Streptomycinsulfat-Fällung (20 mg/ml Endkonzentration, Boehringer) und Reinigung mittels FPLC unter schrittweiser Elution mit KCl über eine Heparin-Sepharose-Säule (Pharmacia). Die Reinheit wurde durch Coomassiefärbung nach SDS-PAGE-Analyse bestimmt, und die Proteinmenge nach dem Bradford-Verfahren (Kit Micro BCA, Pierce) bestimmt. Die FITC-Markierung von EnHD erfolgte wie zuvor beschrieben [Joliot, 1991].

Zur Erzeugung von Hühnchen-En2-Protein, wurde ein 1,1 kb EcoRI-Fragment mit der vollständigen En2-cDNA in die EcoRI-Stelle eines Vektorzwischenproduktes insertiert, das durch Insertion folgender Adapter-Oligonukleotide erhalten wurde:
5'-AGCTTCATATGGAGGAGGGCGGCCGCAGCCCCCGGGAGGAGGAATTCG-3' und
5'-GATCCGAATTCCTCCTCCCGGGGGCTGCGGCCGCCCTCCTCCATATGA-3'
in BamHI- und NdeI-gespaltenen pBluescript. Die 5'-nichtcodierende Sequenz wurde durch Spaltung mit SmaI entfernt. Die En2-Sequenz wurde dann in die NdeI- und BamHI-Stellen des Expressionsvektors pET-3a inseriert.

Das Engrailed2-Protein aus Hühnchen (ChEn2) wurde wie für EnHD beschrieben hergestellt. Eine semigereinigte Fraktion wurde erhalten, indem die Bakterien lysiert wurden und der Extrakt mit Heparin-Sepharose nach [Bourbon, 1995] inkubiert wurde. Die Heparin-bindende Fraktion wurde dann dialysiert und eingeengt (Centriprep, Amicon). ChEn2 wurde durch Western-Blot-Analyse mit 4D9-Antikörper (Patel et al., 1989) identifiziert und für Gelshift-Assays verwendet.

### 5'-RACE

Gesamt-RNA wurde aus ES-C3-Zellen unter Verwendung von Standardverfahren (Kit RNAeasy, Quiagen) isoliert. 200 ng Gesamt-RNA aus den ES-C3-Zellen wurden mittels der reversen Transkriptase "Superscript II" (Gibco/BRL) und einem Primer (5'-TGGCGAAGGGGGATGTGC-3'), der im 5'-Bereich der LacZ-Gensequenzen lokalisiert ist, nach den Angaben des Herstellers revers transkribiert. Ein Zehntel der cDNA wurde mit dCTPs unter Verwendung der terminalen Transferase (Gibco/BRL) mit einem Schwanz versehen. Die cDNA mit C-Schwanz wurde mit einem Primer aus der Spleißakzeptor-Region des Genfallenvektors:
5'-GACTCTGGCGCCGCTGCTCTGTCAG-3' und einem poly-G-Primer:
5'-CUACUACUACUAGGCCACGCGTCGACTAGTACGGIGGGIIGGGIIG-3'
über PCR amplifiziert.

Es wurden dann 1µl der 50-ml-PCR-Reaktion in einer zweiten PCR-Reaktion unter Verwendung eines "nested"-Primers aus der Spleißakzeptorregion: (5'-ACCTGTTGGTCTGAAACTCAGCCT-3') und einem Primer: (5'-CUACUACUACUAGGCCACGCGTCGACTAGTAC-3'), der den 5'-Teil des poly-G-Primers erkennt, verwendet. Die cDNA-Fragmente wurden in das pAMP-Plasmid (GIBCO/BRL) kloniert und gereinigt. Um die Existenz des Fusionstranskriptes zu beweisen, wurden 400 ng Gesamt-RNA aus ES-C3-Zellen mit Hilfe von zufallsgemäßen Hexamerprimern revers transkribiert. Eine erste PCR-Runde erfolgte mit einem stromaufwärts gelegenen Primer aus der *BPAG1n2*-Sequenz
(5'-TGCTGGACCCAGCCGAGAGGGCTGTGC-3') und einem LacZ-Primer
(5'-TGGCGAAAGGGGGATGTGCTGCAAGGCG-3')
und eine zweite PCR-Runde erfolgte mit den "nested"-Primern
(5'-AGCAGATGAGCGGGACAAAGTTCAAAAG-3') und
(5'-CGCCAGGGTTTTCCCAGTCACGACGTTG-3'). Das Fragment wurde isoliert, in pBluescript kloniert und sequenziert.

### Northern-Blot-Analyse und in situ-Hybridisierung.

Zellen wurden über Nacht mit EnHD (300 ng/10⁵ Zellen) in Gegenwart oder Abwesenheit von 10⁻⁶ M Cycloheximid behandelt. Die Northern-Blot- und in situ-Hybridisierung wurden mittels Standard-Techniken [Ausubel, 1994] durchgeführt. Glycerinaldehyd-3-phosphatdehydrogenase (GAPDH) diente als Beladungskontrolle. Die Northern-Blots wurden mit einem Phosphoimager quantifiziert. Zum Nachweis des *BPAG1e*-Transkriptes in Keratinocyten wurde eine Probe aus GMA24FIA-cDNA durch Polymerasekettenreaktion (PCR) isoliert, wobei Taq-Polymerase und die folgenden Oligonukleotidprimer verwendet wurden: 5'-GGAAGTTCTTCCTCTACT-3' und 5'-GGTTGAAACTACTCAGAG-3'. Das entstandene 519 bp große DNA-Amplifikationsprodukt reicht gemäß [Tamai, 1993] von Position 3048 bis Position 3567. Dieses PCR-Produkt wurde mit dem Klenow-Enzym mit stumpfen Enden versehen und in die EcoRV-Stelle von pBluescript kloniert. Die BPAG1n-Isoformen wurden mit einem 664 bp DNA-Fragment nachgewiesen, das der "dt-ABD"-Sonde entspricht und an die Actin-Bindungsdomäne bindet [Bernier, 1995].

### Transfektions-Assays

Zur Expression der Luciferase unter der Kontrolle des *BPAG1e*-Promotors, wurde ein aus dem genomischen Lambda-Zap-Klon (mdt I.4.I.2, von Dr. R. Kothary zur Verfügung gestellt) stammendes 7 kb SacI/SalI-Fragment in pBluescript (Stratagene) subkloniert. Dieses Fragment enthielt die gesamte Intergenregion, was durch Doppelhybridisierung mit *BPAG1e*- und *ABD*-*dt*-Sonden nachgewiesen wurde (nicht gezeigt). Ein 6,5 kb großes BamHI-Fragment wurde dann in die BglII-Stelle von pGL2bas (Promega) inseriert. Schließlich wurde ein 2,3 kb HindIII-Fragment in die HindIII-Stelle von pGL2bas transferiert, so daß pBP-luc erhalten wurde. pBP-luc wurde mit pTL1En2 oder pLT1En2ÆNter in GMA-48 cotransfiziert. Die Kotransfektions-Assays wurden in Suspension mit einem Calciumphosphat-Präzipitat wie in [Le Roux, 1995] beschrieben durchgeführt. Sämtliche Bedingungen wurden vierfach durchgeführt.

### Gelshift und DNase I-Footprinting

DNA-Fragmente wurden mit Polynukleotid-Kinase und (γ-³²P)ATP endmarkiert. Die Extrakte aus EnHD- oder En2-Protein-exprimierenden E. coli und E. coli-Kontrollextrakte wurden zu den markierten Sonden in 20 mM Tris-HCl, pH-Wert 7,9, 35 mM KCl, 2,5 mM MgCl₂, 1 µg poly-dIdC und 15% Glycerin gegeben, 10 min. auf Eis inkubiert und dann auf nativen 6%igen Polyacrylamidgelen analysiert.

Für DNase I-Footprints wurden die Promotorfragmente mit Klenow-Enzym endmarkiert. Die Footprinting-Assays wurden durchgeführt, indem 200 ng Fragment mit EnHD in einem Endvolumen von 50 µl Inkubationsgemisch 20 mM Tris-HCl, pH-Wert 7,9, 35 mM KCl, 2,5 mM MgCl₂, 1 µg poly-dIdC, 4% Polyvinylalkohol und 15% Glycerin 10 min. auf Eis inkubiert wurden, und danach ein gleiches Volumen DNase I in 10 mM MgCl₂, 4 mM CaCl₂ zugegeben wurde. Nach 2minütiger Inkubation wurde die Reaktion durch Zugabe eines gleichen Volumens Stop-Puffers [Ausubel, 1994] beendet, die DNA mit Phenol und Chloroform extrahiert, mit Ethanol gefällt und auf einem 6%igen Sequenziergel analysiert. Oligonukleotidsequenz für Gelshifts: (5'-GAAAAAGTAATTGAACATTTTTCC-3', oberer Strang).
Oligonukleotidsequenzen, die im Gelshift-Assay entsprechend der beiden geschützten Bereiche beim Footprinting verwendet wurden:
(5'-GGCTGGCTCATATGCCTTTATTTTTTTTAATGACTAATTATTTGAG-3', -2222 bis -2176, oberer Strang) und
(5'-CACACTTAGTTACTGAGCCAATATTCTGCG-3', -695 bis -664, oberer Strang).

### LITERATUR

Ausubel, F. M., Brent, R., Kingston, R. E., Moore, D. D., Seidman, J. G., Smith, J. A., and Struhl, K. (1994). Current protocols in molecular biology: Wiley & sons).
Bernier, G., Brown, A., Dalpé, G., De Repentigny, Y., Mathieu, M., and Kothary, R. (1995). Dystonin Expression in the Developing Nervous system Predominates in the Neurons That degenerate in *Dystonia musculorum* Mutant mice. Molecular and cellular neuroscience *6*, 509-520.
Bourbon, H. M., Martin, B. E., Rosen, D., and Kornberg, T. B. (1995). Phosphorylation of the Drosophila engrailed protein at a site outside its homeodomain enhances DNA binding. . Journal of Biological Chemistry *270*, 11130-9.
Joliot, A., Pernelle, C., Deagostini-Bazin, H., and Prochiantz, A. (1991). Antennapedia homeobox peptide regulates neural morphogenesis. Proc. Natl. Acad. Sci. USA *88*, 1864-1868.
Joliot, A. H., Triller, A., Volovitch, M., Pernelle, C., and Prochiantz, A. (1991). α-2, 8-Polysialic acid is the neuronal surface receptor of Antennapedia homeobox peptide. The New Biologist *3*, 1121-1134.
Le Roux, I., Duharcourt, S., Volovotch, M., Prochiantz, A., and Ronchi, E. (1995). Promoter-specific regulation of genes expression by an exogenously added homeodomain that promotes neurite growth. FEBS letters *368*, 311-314.
   1) Hill, D.H.P. and **Wurst, W.**: Mutagenic strategies to dissect mammalian development. Current Topics in Developmental Biology, Volume 28, 181-206, 1993a
   3) Hill, D.H.P. and **Wurst, W.**: Screening for pattern formation genes in mouse using gene trap approaches, Methods in Enzymology, Volume 255, 663-681, 1993b
Tamai, K., Sawamura, D., Do, H. C., Tamai, Y., Li, K., and Uitto, J. (1993). The human 230-kD bullous pemphigoid antigen gene (BPAG1). Exon-intron organization and identification of regulatory tissue specific elements in the promoter region. . Journal of Clinical Investigation *92*, 814-22.
Wurst, W., and Joyner, A. (1993). Production of targeted embryonic stem cell clones. In gene Targeting : apractical approach, A. L. Joyner, ed.

## Patentansprüche

1. Verfahren zur Identifizierung von Transkriptionsfaktor responsiblen Elementen, Zielgenen und/oder Cofaktoren von Transkriptionsfaktoren mit den nachfolgenden Schritten:
(a) Einbringen eines Genfallenvektors in eine eukaryontische Zelle in Kultur, wobei der Genfallenvektor zumindest, in funktioneller Anordnung, die nachfolgenden Elemente aufweist:
- ein Reportergen
- eine Polyadenylierungsstelle
- ein Selektionsmarkergen
(b) Selektion auf Zellen, die den Vektor enthalten;
(c) in Kontakt bringen der selektierten Zellen mit einem Transkriptionsfaktor, dessen zugehöriges Transkriptionsfaktor responsible Element, Zielgen und/oder zugehöriger Cofaktor analysiert werden Soll;
(d) Identifizieren und Kultivieren solcher Zellen, die eine Veränderung der Reportergenaktivität zeigen;
(e) Identifizieren der Zielgene und/oder Cofaktoren der Transkriptionsfaktoren und/oder der Transkriptionsfaktor responsiblen Elemente.

2. Verfahren nach Anspruch 1,
dadurch gekennzeichnet,
daß als Reportergen ein in eukaryontischen Zellen nachweisbares Gen verwendet wird, bevorzugt ausgewählt aus der Gruppe, bestehend aus dem LacZ-Gen, Luciferasegen, Green-Fluoreszenz-Protein-Gen und CAT-Gen.

3. Verfahren nach Anspruch 1 oder 2,
dadurch gekennzeichnet,
daß auf dem Genfallenvektor ein Promotor für das Selektionsmarkergen angeordnet wird.

4. Verfahren nach einem oder mehreren der vorhergehenden Ansprüche,
dadurch gekennzeichnet,
daß der Promotor in embryonalen Stammzellen aktiv ist.

5. Verfahren nach einem oder mehreren der vorhergehenden Ansprüche,
dadurch gekennzeichnet,
daß das Selektionsmarkergen ein Antibiotikumresistenzmarkergen ist, bevorzugt ein Neomyzin- oder Hygromycin-Resistenzgen.

6. Verfahren nach einem oder mehreren der vorhergehenden Ansprüche,
dadurch gekennzeichnet,
daß der Genfallenvektor ein Reportergen zur Negativselektion enthält.

7. Verfahren nach Anspruch 6,
dadurch gekennzeichnet,
daß das Reportergen, bevorzugt ein Thymidinkinasegen, Diphteriegen oder HAT-Gen, gegen Integrationen in konstitutiv aktive Gene selektiert.

8. Verfahren nach einem oder mehreren der vorhergehenden Ansprüche,
dadurch gekennzeichnet,
daß die Selektion auf die den Genfallenvektor enthaltende Zellen dadurch erfolgt, daß die im Schritt (a) erhaltenen Zellen einem Selektionsagens ausgesetzt werden, das für das Selektionsmarkergen spezifisch ist.

9. Verfahren nach Anspruch 8,
dadurch gekennzeichnet,
daß das Agens ein Antibiotikum ist.

10. Verfahren nach einem oder mehreren der vorhergehenden Ansprüche,
dadurch gekennzeichnet,
daß der Transkriptionsfaktor dem Zellkulturmedium der im Schritt (b) erhaltenen Zellen hinzugefügt wird oder daß ein für den Transkriptionsfaktor codierendes Gen in die Zellen eingeführt wird.

11. Verfahren nach Anspruch 10,
dadurch gekennzeichnet,
daß das für den Transkriptionsfaktor codierende Gen auf einem Plasmid liegt und es sich unter der Kontrolle eines induzierbaren Promotors befindet.

12. Verfahren nach einem oder mehreren der vorhergehenden Ansprüche,
dadurch gekennzeichnet,
daß das für den Transkriptionsfaktor kodierende Gen ausgewählt wird aus der Gruppe, umfassend Zinkfingergene, Pou-Gene, Homeobox enthaltende Gene, HGM-Box-enthaltende Gene oder Winged-Helix-Gene.

13. Verfahren nach Anspruch 10 oder 11,
dadurch gekennzeichnet,
daß der Transkriptionsfaktor befähigt ist, die Zellmembran zu passieren.

14. Verfahren nach einem oder mehreren der vorhergenden Ansprüche,
dadurch gekennzeichnet,
daß der Transkriptionsfaktor eine Homeodomäne enthält, die ihn befähigt, die Zellmembran zu passieren.

15. Verfahren nach Anspruch 7,
dadurch gekennzeichnet,
daß die Zellen einem Agens, bevorzugt Gancyclovir, ausgesetzt werden, das selektiv solche Zellen eliminiert, die Integrationen von Genfallenvektoren in konstitutiv aktiven Genen tragen.

16. Verfahren nach einem oder mehreren der vorhergehenden Ansprüche,
dadurch gekennzeichnet,
daß die im Schritt (d) erhaltenen Zellen zur Erzeugung von chimären Säugetieren verwendet werden.

17. Verfahren nach einem oder mehreren der vorhergehenden Ansprüche,
dadurch gekennzeichnet,
daß als Genfallenvektor ein Vektor eingesetzt wird, der, im Leseraster, ein Reportergen , daß an eine Spleißakzeptorstelle fusioniert ist, ein Reportergen, das gegen Integrationen in konstitutiv aktive Gene selektierbar ist, eine interne Ribosomeneintrittstelle sowie ein Selektionsmarkergen, je in funktioneller Verbindung, enthält.


(1) ALLGEMEINE ANGABEN:

(i) ANMELDER:
(A) NAME: GSF-Forschungszentrum für Umwelt und
Gesundheit GmbH
(B) STRASSE: Ingolstädter Landstraße 1
(C) ORT: Neuherberg
(D) BUNDESLAND: Bayern
(E) LAND: Deutschland
(F) POSTLEITZAHL: 85764

(ii) BEZEICHNUNG DER ERFINDUNG: Verfahren zur Identifizierung von
Zielgenen
von Transkriptionsfaktoren

(iii) ANZAHL DER SEQUENZEN: 25

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(v) DATEN DER JETZIGER ANMELDUNG:
ANMELDENUMMER: DE 197 40 578.9

(2) ANGABEN ZU SEQ ID NO: 1:

(i) SEQUENZKENNZEICHEN:
(A) LÄNGE: 47 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 1:

CTCAAATAAT TAGTCATTAA AAAAAATAAA GGCATATGAG CCAGCCT 47

(2) ANGABEN ZU SEQ ID NO: 2:

(i) SEQUENZKENNZEICHEN:
(A) LÄNGE: 30 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 2:

CGCAGAATAT TGGCTCAGTA ACTAAGTGTG 30

(2) ANGABEN ZU SEQ ID NO: 3:

(i) SEQUENZKENNZEICHEN:
(A) LÄNGE: 10 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 3:

TCAATTAAAT 10

(2) ANGABEN ZU SEQ ID NO: 4:

(i) SEQUENZKENNZEICHEN:
(A) LÄNGE: 26 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 4:

TTTCATATGG AAGACAAGCG GCCCCG 26

(2) ANGABEN ZU SEQ ID NO: 5:

(i) SEQUENZKENNZEICHEN:
(A) LÄNGE: 32 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 5:

GGGGGATCCC TACGCCTTCT TGATCTTGGC TC 32

(2) ANGABEN ZU SEQ ID NO: 6:

(i) SEQUENZKENNZEICHEN:
(A) LÄNGE: 48 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 6:

AGCTTCATAT GGAGGAGGGC GGCCGCAGCC CCCGGGAGGA GGAATTCG 48

(2) ANGABEN ZU SEQ ID NO: 7:

(i) SEQUENZKENNZEICHEN:
(A) LÄNGE: 48 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 7:

GATCCGAATT CCTCCTCCCG GGGGCTGCGG CCGCCCTCCT CCATATGA 48

(2) ANGABEN ZU SEQ ID NO: 8:

(i) SEQUENZKENNZEICHEN:
(A) LÄNGE: 18 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 8:

TGGCGAAGGG GGATGTGC 18

(2) ANGABEN ZU SEQ ID NO: 9:

(i) SEQUENZKENNZEICHEN:
(A) LÄNGE: 25 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 9:

GACTCTGGCG CCGCTGCTCT GTCAG 25

(2) ANGABEN ZU SEQ ID NO: 10:

(i) SEQUENZKENNZEICHEN:
(A) LÄNGE: 46 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 10:

CUACUACUAC UAGGCCACGC GTCGACTAGT ACGGRGGGRR GGGRRG 46

(2) ANGABEN ZU SEQ ID NO: 11:

(i) SEQUENZKENNZEICHEN:
(A) LÄNGE: 24 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 11:

ACCTGTTGGT CTGAAACTCA GCCT 24

(2) ANGABEN ZU SEQ ID NO: 12:

(i) SEQUENZKENNZEICHEN:
(A) LÄNGE: 32 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 12:

CUACUACUAC UAGGCCACGC GTCGACTAGT AC 32

(2) ANGABEN ZU SEQ ID NO: 13:

(i) SEQUENZKENNZEICHEN:
(A) LÄNGE: 27 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 13:

TGCTGGACCC AGCCGAGAGG GCTGTGC 27

(2) ANGABEN ZU SEQ ID NO: 14:

(i) SEQUENZKENNZEICHEN:
(A) LÄNGE: 28 Basenpaare
(B) ART: Nucleotid
(C) STRANGFORM: Doppelstrang

(D) TOPOLOGIE: linear

(ii) ART DES MOLEKÜLS: Genom-DNA

(xi) SEQUENZBESCHREIBUNG: SEQ ID NO: 14:

TGGCGAAAGG GGGATGTGCT GCAAGGCG 28

(2) ANGABEN ZU SEQ ID NO: 15:

(i) SEQUENZKENNZEICHEN:
(A) LÄNGE: 28 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 15:

AGCAGATGAG CGGGACAAAG TTCAAAAG 28

(2) ANGABEN ZU SEQ ID NO: 16:

(i) SEQUENZKENNZEICHEN:
(A) LÄNGE: 28 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 16:

CGCCAGGGTT TTCCCAGTCA CGACGTTG 28

(2) ANGABEN ZU SEQ ID NO: 17:

(i) SEQUENZKENNZEICHEN:
(A) LÄNGE: 18 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 17:

GGAAGTTCTT CCTCTACT 18

(2) ANGABEN ZU SEQ ID NO: 18:

(i) SEQUENZKENNZEICHEN:
(A) LÄNGE: 18 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 18:

GGTTGAAACT ACTCAGAG 18

(2) ANGABEN ZU SEQ ID NO: 19:

(i) SEQUENZKENNZEICHEN:
(A) LÄNGE: 24 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 19:

GAAAAAGTAA TTGAACATTT TTCC 24

(2) ANGABEN ZU SEQ ID NO: 20:

(i) SEQUENZKENNZEICHEN:
(A) LÄNGE: 46 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 20:

GGCTGGCTCA TATGCCTTTA TTTTTTTTAA TGACTAATTA TTTGAG 46

(2) ANGABEN ZU SEQ ID NO: 21:

(i) SEQUENZKENNZEICHEN:
(A) LÄNGE: 30 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 21:

CACACTTAGT TACTGAGCCA ATATTCTGCG 30

(2) ANGABEN ZU SEQ ID NO: 22:

(i) SEQUENZKENNZEICHEN:
(A) LÄNGE: 225 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 22:

TGCTGGACCC AGCCGAGAGG GCTGTGCTTC GGATAGCAGA TGAGCGGGAC AAAGTTCAAA 60

AGAAAACATT TACAAAATGG ATAAATCAGC ATCTCATGAA GGGTCCCAGG TCCCGAAAAC 120

CAAAGAAGAA GAACCCTAAC AAAGAGGACA AGCGGCCTCG CACAGCCTTC ACTGCTGAGC 180

AGCTCCAGAG GCTCAAGGCT GAGTTTCAGA CCAACAGGTC GACAA 225

(2) ANGABEN ZU SEQ ID NO: 23:

(i) SEQUENZKENNZEICHEN:
(A) LÄNGE: 72 Aminosäuren
(B) ART: Aminosäure
(C) STRANGFORM: Einzelstrang
(D) TOPOLOGIE: linear

(ii) ART DES MOLEKÜLS: Protein

(xi) SEQUENZBESCHREIBUNG: SEQ ID NO: 23:

Leu Asp Pro Ala Glu Arg Ala Val Leu Arg Ile Ala Asp Glu Arg Asp
1               5                   10                  15

Lys Val Gln Lys Lys Thr Phe Thr Lys Trp Ile Asn Gln His Leu Met
            20                  25                  30

Lys Gly Pro Arg Ser Arg Lys Pro Lys Lys Lys Asn Pro Asn Lys Glu
        35                  40                  45

Asp Lys Arg Pro Arg Thr Ala Phe Thr Ala Glu Gln Leu Gln Arg Leu
    50                  55                  60

Lys Ala Glu Phe Gln Thr Asn Arg
65                  70

(2) ANGABEN ZU SEQ ID NO: 24:

(i) SEQUENZKENNZEICHEN:
(A) LÄNGE: 657 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 24:

CTGAATTGCA ACCTGCCCTG CCCCTTGTTT AGCTTCCCAA GTACTGGGCT TACAGTAGAC 60

TTGCACTGTA ACACAAGCCT AGATAATAAA CTTCTTTTGT GATATGTATG CAGTCTGTAA 120

CAATACAAAG TGGATTATGA TGCACATTAA AATTCTGATA CTGCTGTAGT GACCAAAACT 180

ATCTTTTCTT GGTGTCTTTA ATTCAAATGG CTCATTTAAT GCTTCATTTT AACTACCTGT 240

AAGGGCTGCA CCCTAAGCAT TTACAGTTCA TTTTAGTTCA TAGGCTGCTT ATGTAGACAT 300

GGTAACTTTA TGAGTTAACA AGGATGCAGC CAGTAATAAA GGGTGGACTT GCCTTTGCAT 360

TCATAATGTA ATATATTATC TTTAATTCAT TACTCCATGG CAACACTGAA GTCGGTGTCT 420

AAGACAGTGA GGACACAGCT ACACTGCGTG TCTCTGTTTT GTGCTTGGCA TTGAGCAAAG 480

GCACTGGTAA GGGACCCAAA ATGATAGCTC CTCTCTACAT TCTCAGAGCG GATACCTAAG 540

TAACAAGTGG CTTAAACACT CCATGAAGAA ATATGAAATT ATAACCAAGG AAAGAACCCT 600

GAACAATACT CAAATAATTA GTCATTAAAA AAAATAAAGG CATATGAGCC AGCCTGG 657

(2) ANGABEN ZU SEQ ID NO: 25:

(i) SEQUENZKENNZEICHEN:
(A) LÄNGE: 639 Basenpaare
(B) ART: Nucleotid
(C) STRANGFORM: Doppelstrang
(D) TOPOLOGIE: linear

(ii) ART DES MOLEKÜLS: Genom-DNA

(xi) SEQUENZBESCHREIBUNG: SEQ ID NO: 25:

CTTAAATATG CTGTGGCGTG ATATGCAATG CACTGCAAAT ACTATGCTGA TAACTATTCT 60

AAAGAATCCT GCCTACAGCC ATTCATTTGC TTGCTGAGGT TTGCCTTTTG TAGCCAGCAT 120

TAAAGCCCTG CAAGTACGGG CAGAGCAGGA CGATTGGGCG TCACTGGGAA AGATATGCCA 180

AATATTTTCA CCAAGAGTCA TCCAGCTTCA ACCACCTAAA AAACAACTCA GGTACACAGG 240

AGCCCGGGCA GCACCAGTGA AGGTAATGCT ATCAATACCA AGCACCAACC AGAAACACAA 300

AGCTCAGATC ACACAGCTGG ACAAATGCTG GACAGCAAGC AGATCCCACG TGCTTCCTAA 360

CACAAGGTAA AATGCTGGAC TAATCAATAA GTCAATAATC AATAAGCAAG CACACAAATT 420

TAATGAGAAA CCAAATCTGT TTCCATTTGA CCATCTCTTC CATTGTTCAC CGCATTAAAT 480

TAACTGGAAA AATGTTCAAT TACTTTTTCT TTGTGTGATA AACTCTCTCT CCTAAGCCAC 540

TACTGTTTGG ATTCTAAAAG CCAAAGACTC CACGCAGAAT ATTGGCTCAG TAACTAAGTG 600

TGAATGTGTC TGGGCTTGCT TCTTCTTCAC ACAGACTAG 639